# EUROPEAN PATENT SPECIFICATION

(11) **EP 4 405 047 B1**
(45) Date of publication and mention of the grant of the patent: **01.04.2026**
(21) Application number: 22782447.1
(22) Date of filing: 07.09.2022
(51) Int. Cl.: A61K 9/20, A61K 9/14, A61P 25/00, A61P 25/04, A61P 29/00, A61K 31/05, A61K 31/352, A61K 9/48

(54) **CANNABINOID FORMULATION FOR ORAL ADMINISTRATION**
CANNABINOIDFORMULIERUNG ZUR ORALEN VERABREICHUNG
FORMULATION DE CANNABINOÏDE POUR ADMINISTRATION ORALE

(30) Priority: 22.09.2021 CZ 20210448
(43) Date of publication of application: 31.07.2024
(73) Proprietor: CB21 Pharma, S.R.O., 61200 Brno (CZ)
(72) Inventor: MALIK, Antonin, 62100 Brno (CZ); STORCH, Jan, 10000 Praha 10 (CZ)
(74) Representative: Harber IP s.r.o.
(86) International application number: PCT/CZ2022/050086
(87) International publication number: WO 2023/046220

(56) References cited:
- WO-A1-2020/024011
- WO-A1-2020/222029
- WO-A1-2020/234675
- US-A1- 2020 330 425

## Description

### Field of Art

The present invention relates to an enterosolvent formulation of cannabinoids suitable for oral administration, with an improved dissolution profile and delayed release of active substances.

### Background Art

Cannabis plant secondary metabolites, cannabinoids, are known to have pharmacological effects. The main representative is Δ9-tetrahydrocannabinol (THC), the intake of which leads to the modulation of the endocannabinoid system and is manifested by a psychotropic effect. THC is the reason why cannabis flowers are used (or abused) for their narcotic effects as a recreational drug. Cannabis, however, contains more than 100 phytocannabinoids, the best-known representative of which is cannabidiol (CBD), which belongs to the group of non-psychotropic phytocannabinoids, cannabinol (CBN), cannabichromene (CBC), cannabigerol (CBG), cannabivarin, and their derivatives or analogues. Cannabidiol, cannabigerol and cannabinol have antioxidant, analgesic, anti-inflammatory, antitumor, antispastic, anxiolytic, antiemetic, anticonvulsant, neuroprotective, anti-diabetic effects, reduce anxiety, reduce intraocular pressure and drainage of intraocular fluid, improve cognitive functions and improve sleep quality.

Cannabinoids are lipophilic substances (log P is 6 to 7) with very low solubility in water, they are prone to degradation, especially in solution under the influence of light, atmospheric oxygen and acidic pH. The bioavailability of active substances administered orally depends primarily on the extent to which they are absorbed in the gastrointestinal tract. Lipophilic substances are generally poorly absorbed, mainly due to their poor solubility and/or dispersibility in water. The bioavailability of an orally used active substance also depends on the susceptibility of the substance to the first pass effect. Substances absorbed from the intestine must first pass through the liver, where they are metabolized, before being distributed to the system. It is generally believed that cannabinoids are largely metabolized in the liver and their oral bioavailability is low (S. Zhornitsky, S. Potvin, Pharmaceuticals (2012) 5, 529-552). The formulation of cannabinoids therefore plays a key role in increasing their solubility, chemical stability and residence time in the gastrointestinal tract.

A widely used administration route is oral administration in the solid state, which allows 100% of the drug to enter the digestive tract. Oral solid formulations currently under investigation include Columbia Care's CBD pressed tablets called BeneCeed. Administration in this manner ensures a consistent dose, but does not necessarily solve problems associated with poor bioavailability. Echo Pharmaceuticals and Ananda Scientific are researching products that purportedly increase bioavailability by increasing CBD's solubility in water (Ananda's drops or gel capsules and Echo Pharmaceuticals' Arvisol compressed tablets). Gelatin capsules developed by Lexaria Bioscience Corp called TurboCBD are used in dietary supplements. Another pharmaceutical form is cannabidiol encapsulated in polymeric particles in the form of a lyophilized powder (manufactured by Aphios) whose function is sustained release.

US 2020/330425 discloses a lozenge formulation for improved mucosal delivery of cannabinoids. Traditional methods such as smoking or oral oils face challenges including poor bioavailability, undesirable taste, and limited dosing efficiency. The disclosure addresses these issues by combining a mucosal delivery enhancing component with extragranular sugar alcohols, enabling higher cannabinoid loading, better sensory properties, and enhanced absorption through the oral mucosa.

### Disclosure of the Invention

The present invention overcomes the above described problems by providing a pharmaceutical formulation with good bioavailability of effective cannabinoids and with their delayed release. The formulation also enables the stabilization of the cannabinoids, maximizing their concentration on the mucosa and improving their dispersibility in the aqueous environment. Furthermore, in some embodiments, the formulation overcomes the problems associated with susceptibility to the first pass effect by the fact that the formulation is enterosolvent, i.e. the delayed release occurs in the small intestine.

The pharmaceutical formulation according to the present invention contains a particulate porous sorbent onto which is applied a mixture of at least one cannabinoid selected from cannabidiol, cannabigerol, cannabinol, D9-THC and D8-THC with a nonionic surfactant.

The cannabinoids contained in the mixture with the nonionic surfactant may be in the form of a cannabis extract.

The particulate sorbent is selected from the group comprising aluminosilicates and their salts, with a particle size in the range of 50 to 800 µm, and preferably with a neutral or basic pH. The particle sorbent is most preferably magnesium aluminosilicate, more preferably with a particle size in the range of 50 to 200 µm.

The nonionic surfactant is polyoxyethylene sorbitan monooleate (polysorbate 80).

Preferably, the pharmaceutical formulation contains 20 to 60 wt. % of the particulate sorbent, 20 to 50 wt. % of the surfactant, and 20 to 50 wt. % of at least one cannabinoid selected from cannabidiol, cannabigerol, cannabinol, D9-THC and D8-THC.

A particularly preferred formulation contains 40 to 60 wt. % of the particulate sorbent, 20 to 40 wt. % of the surfactant, and 20 to 40 wt. % of at least one cannabinoid selected from cannabidiol, cannabigerol, cannabinol, D9-THC and D8-THC.

Most preferably, the formulation contains 46 to 64 wt. % of the particulate sorbent, 23 to 27 wt. % of the surfactant, and 23 to 27 wt. % of at least one cannabinoid selected from cannabidiol, cannabigerol, cannabinol, D9-THC and D8-THC.

In a particularly preferred embodiment, the formulation contains 50 wt. % of the particulate sorbent, 25 wt. % of the surfactant and 25 wt. % of at least one cannabinoid selected from cannabidiol, cannabigerol, cannabinol, D9-THC and D8-THC.

In a particularly preferred embodiment of the invention, the weight ratio of cannabinoid: polysorbate 80: particulate sorbent is 1: 1: 1 to 1: 1:2, or 1: 1: 1 to 1:2:2.

The pharmaceutical formulation may further contain one or more additional active ingredients or auxiliary active substances, in particular selected from rosmarinic acid (which is typically contained in plant extracts, for example in lemon balm extract), ginkgolide A, ginkgolide B, bilobalide (these are typically contained in the extract of ginkgo biloba), polyphenols and terpenes from hops and hemp extract, and plant extracts containing these substances, germacrone and turmerones from turmeric, phytosterols and terpenoids, kynurenic acid and its derivatives, curcumin, vitamins, antioxidants.

The additional active ingredients or auxiliary active substances may be included in the formulation in an amount of up to 20 wt. %, preferably up to 10 wt. %, even more preferably up to 5 wt. %, and most preferably up to 2 wt. %.

In one preferred embodiment, the auxiliary active substance is lemon balm extract containing rosmarinic acid.

In one particularly preferred embodiment, the formulation contains 45.5 wt. % of the particulate sorbent, 22.7 wt. % of the surfactant, 22.7 wt. % of at least one cannabinoid selected from cannabidiol, cannabigerol, cannabinol, D9-THC and D8-THC and 9.1 wt. % of lemon balm extract with a minimum content of 2 wt. % rosmarinic acid.

The pharmaceutical formulation is preferably filled into capsules. In particular, hard capsules, acid-resistant capsules, enteric capsules or soft gelatin capsules can be used.

In some embodiments, the pharmaceutical formulation may be mixed with pharmaceutically acceptable excipients and formulated into tablets or coated tablets. Coated tablets may have an acid-resistant or enteric coating. Pharmaceutically acceptable excipients include, in particular, fillers, binders and/or disintegrants. Particularly preferred pharmaceutically acceptable excipients are magnesium stearate, sodium or calcium carboxymethylcellulose, and/or polyvinylpyrrolidone.

Another object of the present invention is a method of preparing a pharmaceutical formulation, comprising the steps of:
a) dissolving at least one cannabinoid selected from cannabidiol, cannabigerol, cannabinol, D9-THC and D8-THC, optionally together with lipophilic additional active ingredients or auxiliary active substances in polysorbate 80,
b) applying the mixture prepared in step a) to the particulate porous carrier,
c) homogenization of the product of step b),
d) optional mixing of the product of step c) with hydrophilic additional active ingredients or auxiliary active substances.

Furthermore, the pharmaceutical formulation can optionally be mixed with pharmaceutically acceptable excipients, and subsequently filled into capsules, or formulated into tablets or coated tablets.

Preferably, step a) is carried out at a temperature in the range of 10 to 60°C, more preferably 40 to 60°C.

Step c) is preferably carried out by homogenization in a paddle drum. Homogenization in a paddle drum can be carried out at room temperature.

Step d) is preferably performed by mixing the mixture from step c) with additional active ingredients or auxiliary active substances, and subsequently homogenizing the mixture, preferably in a paddle drum.

The pharmaceutical formulation according to the invention is suitable for use as a medicament, in particular for use in the prevention and treatment of anxiety disorders and sleep disorders, for the treatment of schizophrenia, post-traumatic stress disorders, Parkinson's disease, Alzheimer's disease, paraphrenia, glaucoma, suppression of inflammation, suppression of acute/chronic/neuropathic pain, treatment of immune-related diseases, diabetes, epilepsy, idiopathic intestinal inflammation including Crohn's disease, treatment of cardiovascular diseases including treatment of high blood pressure, treatment of gastrointestinal disorders and diseases, treatment of inflammation of the prostate and conditions after removal of the prostate, as an auxiliary antitumor treatment, in the reduction of oxidative stress, to improve sleep quality, to improve cognitive functions, and as antibacterial, antiviral and/or neuroprotective drugs.

The pharmaceutical formulation according to the invention containing rosmarinic acid is particularly suitable for use in the prevention or treatment of anxiety, to improve cognitive functions and/or to improve sleep quality.

### Brief Description of Drawings

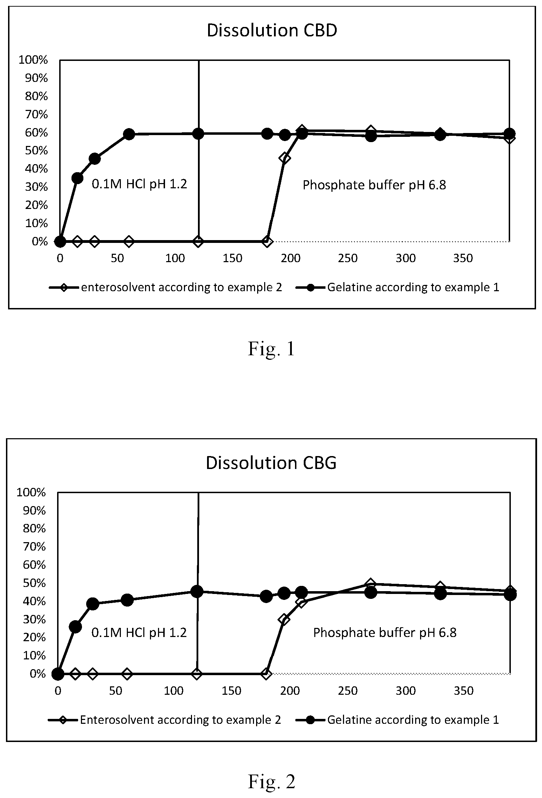

Fig. 1 shows the dissolution profiles of cannabidiol formulations (Example 9).
Fig. 2 shows the dissolution profiles of cannabigerol formulations (Example 9).
Fig. 3 shows the dissolution profiles of cannabinol formulations (Example 9).
Fig. 4 shows the dissolution profiles of the formulation of the invention and two prior art formulations (Example 10).

### Examples of carrying out the Invention

### Example 1 - comparative example

The composition expressed as the composition of one capsule is as follows:

| | |
|---|---|
| Microcrystalline cellulose | 170.0 mg |
| Cannabidiol (CBD) | 50.0 mg |

Both components are mixed in a drum homogenizing device for 20 minutes and then filled into gelatin capsules.

### Example 2:

Capsules and tablets with various cannabinoids have been prepared. The composition expressed as the composition of one capsule /tablet is as follows:

| | |
|---|---|
| Magnesium aluminosilicate (Neusilin) | 100.0 mg |
| Polysorbate 80 | 50.0 mg |
| Cannabidiol or Cannabigerol or Cannabinol or D9-THC or D8-THC | 50.0 mg |
| Lemon balm extract containing rosmarinic acid | 20.0 mg |

Cannabidiol or cannabigerol or cannabinol is dissolved in polysorbate 80 at a temperature not exceeding 60°C and the resulting solution is poured into a drum homogenization device with a Neusilin sorbent. Both components are mixed in the drum homogenizer for 20 minutes. Subsequently, solid lemon balm extract is added and the mixture is mixed for 20 minutes. The resulting mixture is filled into acid-resistant capsules, gelatin capsules, or is pressed into tablets.

### Examples 3-8:

The preparation procedure was the same as in Example 2, other extracts could be added instead of lemon balm extract. Examples of composition of the formulations are shown in the table:

| | Example 3 | Example 4 | Example 5 | Example 6 | Example 7 | Example 8 |
|---|---|---|---|---|---|---|
| Magnesium aluminosilicate | 50 | 100 | 100 | 100 | 100 | 200 |
| Polysorbate 80 | 50 | 50 | 50 | 100 | 100 | 100 |
| CBD, CBG, CBN, D9-THC or D8-THC or cannabis extract | 50 | 50 | 50 | 50 | 100 | 100 |
| Lemon balm extract | 0 | 0 | 0 | 0 | 0 | 0 |
| Hops extract | 0 | 50 | 0 | 0 | 0 | 0 |
| Curcuma-turmerona extract | 0 | 0 | 20 | 0 | 0 | 0 |

### Example 9: Dissolution tests

To determine the release of cannabidiol | cannabigerol | cannabinol from the formulations according to examples 1 and 2, corresponding to a dose of 50 mg of cannabinoid, the cup dissolution method was used. The temperature of the medium was 37±0.5 °C, the rotational speed of the cup was 100 rpm. Capsules were first placed in 0.1 M hydrochloric acid, pH 1.2 (0 to 120 min, simulating the stomach environment). After that, the dissolution medium was changed to a phosphate buffer of pH 6.8 (120 to 300 min, simulating the environment of the small intestine). As part of the experiments, acid-resistant (enteric) capsules and gelatin capsules containing the formulation according to example 2 were compared with gelatin capsules containing the formulation according to example 1.

The dissolution of gelatin capsules containing the formulation according to example 1 provides a similar value for all three cannabinoids. Only up to 0.5% of the original amount of cannabinoids is released from the formulation.

In contrast, the formulation according to example 2 releases an average of 40 to 60% of the original amount of cannabinoids into the medium. The results show that the pharmaceutical formulation according to the invention leads to a significant improvement in the release and dispersibility of cannabinoids in the gastrointestinal tract (GIT) environment and can also be used in the enteric version, i.e. for delayed release of cannabinoids in the small intestine. This partially solves the disadvantages associated with susceptibility to the first pass effect.

### Example 10: Comparison of the invention with prior art formulations

The formulation of Example 2 was compared with formulations according to two prior art documents - WO 2019/135225 (D1) and WO 220/024011 (D2).

The comparative formulations were as follows:

| Composition D1 (Example 6A) | |
|---|---|
| Component | wt. % |
| CBD | 30 |
| Tocopheryl PEG 1000 | 6 |
| Succinate | |
| PEG 35 Castor Oil | 4 |
| Sucrose Stearate | 4 |
| Sorbitan Laurate | 4 |
| Neusilin | 46 |
| Silica | 6 |

| Composition D2 (Example 4) | |
|---|---|
| Component | wt. % |
| CBD | 7.5 |
| MCT Oil | 14.9 |
| Polyoxyl Castor Oil (PEG 35 Castor Oil) | 4.5 |
| Silica | 22.2 |
| Microcrystalline Cellulose | 34.9 |
| Ca₃(PO₄)₂ | 15.9 |

The basket dissolution method was used to compare the release of cannabidiol from the formulations according to example 2 of the present application and the formulations according to D1 and D2. The temperature of the medium was 37±0.5 °C, the rotational speed of the cup was 100 rpm. The individual formulations were placed in a phosphate buffer with a pH of 6.8 (simulation of the environment of the small intestine). At time intervals ranging from 0 to 220 min (0, 15, 30, 60, 120, 180, 220 min), samples were taken and the concentration of CBD in the medium was determined by HPLC. After each sample collection, the buffer was replenished to the original volume.

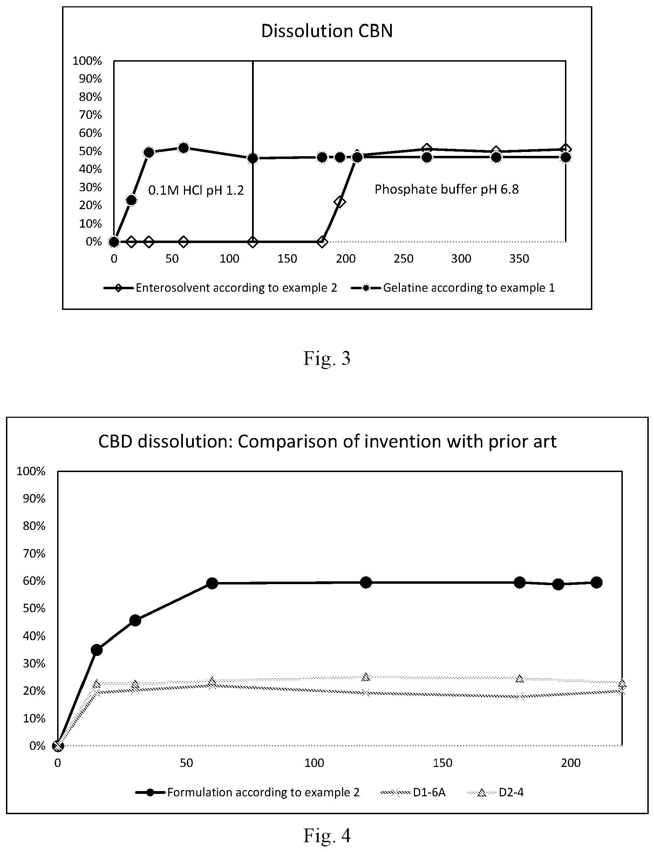

The results are shown in Fig. 4. The formulation according to example 2 releases an average of 60% of the original amount of CBD into the medium, while the formulations according to D1 and D2 only release about 20% of the original amount of CBD. Such a large difference in dissolution behavior is very surprising.

## Claims

1. Pharmaceutical formulation, **characterized in that** it contains a particulate porous sorbent selected from the group comprising aluminosilicates and their salts, with a particle size in the range of 50 to 800 µm, on which a mixture of polyoxyethylene sorbitan monooleate with at least one cannabinoid selected from cannabidiol, cannabigerol, cannabinol, D9-THC and D8-THC is applied.

2. Pharmaceutical formulation according to claim 1, wherein the particle sorbent is magnesium aluminosilicate, preferably with a particle size in the range of 50 to 200 µm.

3. Pharmaceutical formulation according to any one of claims 1 to 2, which contains 20 to 60 wt. % of the particulate porous sorbent, 20 to 50 wt. % of polyoxyethylene sorbitan monooleate, and 20 to 50 wt. % of at least one cannabinoid selected from cannabidiol, cannabigerol, cannabinol, D9-THC and D8-THC.

4. Pharmaceutical formulation according to any one of claims 1 to 2, wherein the weight ratio of cannabinoid(s) : polyoxyethylene sorbitan monooleate : particulate porous sorbent in the formulation is 1: 1: 1 to 1: 1:2, or 1: 1: 1 to 1:2:2.

5. Pharmaceutical formulation according to any one of claims 1 to 4, which further contains one or more additional active ingredients or auxiliary active substances selected from rosmarinic acid, ginkgolide A, ginkgolide B, bilobalide, polyphenols and terpenes from hop extract and hemp, and plant extracts containing these substances, germakrone and turmerones from turmeric, phytosterols and terpenoids, kynurenic acid and its derivatives, curcumin, vitamins, and antioxidants.

6. Pharmaceutical formulation according to claim 5, wherein the additional active ingredients or auxiliary active substances are contained in the formulation in an amount of up to 20 wt. %, preferably up to 10 wt. %, even more preferably up to 5 wt. %, and most preferably up to 2 wt. %.

7. Pharmaceutical formulation according to any one of claims 1 to 6, which is formulated into hard capsules, acid-resistant capsules, enteric capsules, soft gelatin capsules, tablets or coated tablets.

8. A method of preparing the pharmaceutical formulation according to any one of claims 1-7, comprising the steps of:
a) dissolving at least one cannabinoid selected from cannabidiol, cannabigerol, cannabinol, D9-THC and D8-THC, optionally together with lipophilic additional active ingredients or auxiliary active substances in polyoxyethylene sorbitan monooleate,
b) applying the mixture prepared in step a) to the particulate porous sorbent,
c) homogenization of the product of step b),
d) optional mixing of the product of step c) with hydrophilic additional active ingredients or auxiliary active substances.

9. Pharmaceutical formulation according to any one of claims 1 to 7 for use as a medicament.

10. Pharmaceutical formulation according to any one of claims 1 to 7 for use for the prevention and treatment of anxiety disorders and sleep disorders, for the treatment of schizophrenia, post-traumatic stress disorders, Parkinson's disease, Alzheimer's disease, paraphrenia, glaucoma, suppression inflammation, suppression of acute/chronic/neuropathic pain conditions, treatment of immune-related diseases, diabetes, epilepsy, idiopathic intestinal inflammation including Crohn's disease, treatment of cardiovascular diseases including treatment of high blood pressure, treatment of gastrointestinal disorders and diseases, treatment of prostate inflammation and conditions after prostate removal, as an auxiliary anti-tumor treatment, in the reduction of oxidative stress, to improve the quality of sleep, to improve cognitive effects, and as antibacterial, antiviral and neuroprotective drugs.

## Patentansprüche

1. Pharmazeutische Formulierung, **dadurch gekennzeichnet, dass** sie ein partikuläres poröses Sorptionsmittel, ausgewählt aus der Gruppe der Aluminosilikate und ihrer Salze, mit einer Partikelgröße im Bereich von 50 bis 800 µm enthält, auf das eine Mischung von Polyoxyethylensorbitanmonooleat mit mindestens einem Cannabinoid, ausgewählt aus Cannabidiol, Cannabigerol, Cannabinol, D9-THC und D8-THC, aufgebracht ist.

2. Pharmazeutische Formulierung nach Anspruch 1, wobei das partikuläre Sorptionsmittel Magnesiumaluminosilicat ist, vorzugsweise mit einer Partikelgröße im Bereich von 50 bis 200 µm.

3. Pharmazeutische Formulierung nach einem der Ansprüche 1 oder 2, die 20 bis 60 Gew.-% des partikulären porösen Sorptionsmittels, 20 bis 50 Gew.-% Polyoxyethylensorbitanmonooleats und 20 bis 50 Gew.-% mindestens eines Cannabinoids, ausgewählt aus Cannabidiol, Cannabigerol, Cannabinol, D9-THC und D8-THC, enthält.

4. Pharmazeutische Formulierung nach einem der Ansprüche 1 oder 2, wobei das Gewichtsverhältnis von Cannabinoid(en) : Polyoxyethylensorbitanmonooleat : partikulärem porösem Sorptionsmittel in der Formulierung 1:1:1 bis 1:1:2 oder 1:1:1 bis 1:2:2 beträgt.

5. Pharmazeutische Formulierung nach einem der Ansprüche 1 bis 4, die ferner einen oder mehrere zusätzliche Wirkstoffe oder Hilfsstoffe enthält, ausgewählt aus Rosmarinsäure, Ginkgolid A, Ginkgolid B, Bilobalid, Polyphenolen und Terpenen aus Hopfenextrakt und Hanf sowie Pflanzenextrakten, die diese Stoffe enthalten, Germakrone und Turmerone aus Kurkuma, Phytosterolen und Terpenoiden, Kynurensäure und deren Derivaten, Curcumin, Vitaminen und Antioxidantien.

6. Pharmazeutische Formulierung nach Anspruch 5, wobei die zusätzlichen Wirkstoffe oder Hilfsstoffe in der Formulierung in einer Menge von bis zu 20 Gew.-%, vorzugsweise bis zu 10 Gew.-%, noch bevorzugter bis zu 5 Gew.-% und am meisten bevorzugt bis zu 2 Gew.-% enthalten sind.

7. Pharmazeutische Formulierung nach einem der Ansprüche 1 bis 6, formuliert zu Hartkapseln, säurebeständigen Kapseln, magensaftresistenten Kapseln, Weichgelatinekapseln, Tabletten oder Filmtabletten.

8. Verfahren zur Herstellung der pharmazeutischen Formulierung nach einem der Ansprüche 1 bis 7, umfassend die folgenden Schritte:
a) Lösen mindestens eines Cannabinoids, ausgewählt aus Cannabidiol, Cannabigerol, Cannabinol, D9-THC und D8-THC, gegebenenfalls zusammen mit lipophilen zusätzlichen Wirkstoffen oder Hilfsstoffen, in Polyoxyethylensorbitanmonooleat,
b) Auftragen der in Schritt a) hergestellten Mischung auf das partikuläre poröse Sorptionsmittel,
c) Homogenisieren des Produkts aus Schritt b),
d) gegebenenfalls Mischen des Produkts aus Schritt c) mit hydrophilen zusätzlichen Wirkstoffen oder Hilfsstoffen.

9. Pharmazeutische Formulierung nach einem der Ansprüche 1 bis 7 zur Verwendung als Arzneimittel.

10. Pharmazeutische Formulierung nach einem der Ansprüche 1 bis 7 zur Verwendung bei der Vorbeugung und Behandlung von Angststörungen und Schlafstörungen, bei der Behandlung von Schizophrenie, posttraumatischen Belastungsstörungen, Parkinson-Krankheit, Alzheimer-Krankheit, Paraphrenie, Glaukom, zur Unterdrückung von Entzündungen, zur Unterdrückung von akuten/chronischen/neuropathischen Schmerzzuständen, zur Behandlung von immunbedingten Erkrankungen, Diabetes, Epilepsie, idiopathischer Darmentzündung einschließlich Morbus Crohn, zur Behandlung von Herz-Kreislauf-Erkrankungen einschließlich der Behandlung von Bluthochdruck, zur Behandlung von Magen-Darm-Erkrankungen und -Beschwerden, zur Behandlung von Prostataentzündungen und Zuständen nach Prostataentfernung, als unterstützende Antitumorbehandlung, zur Reduzierung von oxidativem Stress, zur Verbesserung der Schlafqualität, zur Verbesserung der kognitiven Funktionen sowie als antibakterielles, antivirales und neuroprotektives Arzneimittel.

## Revendications

1. Formulation pharmaceutique, **caractérisée en ce qu'**elle contient un sorbant poreux particulaire choisi parmi les aluminosilicates et leurs sels, d'une granulométrie comprise entre 50 et 800 µm, sur lequel est appliqué un mélange de monooléate de sorbitan polyoxyéthyléné et d'au moins un cannabinoïde choisi parmi le cannabidiol, le cannabigérol, le cannabinol, le D9-THC et le D8-THC.

2. Formulation pharmaceutique selon la revendication 1, dans laquelle le sorbant particulaire est un aluminosilicate de magnésium, de préférence d'une granulométrie comprise entre 50 et 200 µm.

3. Formulation pharmaceutique selon l'une quelconque des revendications 1 à 2, contenant de 20 à 60 % en poids de sorbant poreux particulaire, de 20 à 50 % en poids de monooléate de sorbitan polyoxyéthyléné et de 20 à 50 % en poids d'au moins un cannabinoïde choisi parmi le cannabidiol, le cannabigérol, le cannabinol, le D9-THC et le D8-THC.

4. Formulation pharmaceutique selon l'une quelconque des revendications 1 à 2, dans laquelle le rapport pondéral cannabinoïde(s) : monooléate de sorbitan polyoxyéthyléné : sorbant poreux particulaire dans la formulation est de 1:1:1 à 1:1:2, ou de 1:1:1 à 1:2:2.

5. Formulation pharmaceutique selon l'une quelconque des revendications 1 à 4, contenant en outre un ou plusieurs principes actifs ou substances actives auxiliaires supplémentaires choisis parmi l'acide rosmarinique, le ginkgolide A, le ginkgolide B, le bilobalide, les polyphénols et les terpènes d'extraits de houblon et de chanvre, et les extraits végétaux contenant ces substances, la germakrone et les turmérones du curcuma, les phytostérols et les terpénoïdes, l'acide kynurénique et ses dérivés, la curcumine, les vitamines et les antioxydants.

6. Formulation pharmaceutique selon la revendication 5, dans laquelle les principes actifs ou substances actives auxiliaires supplémentaires sont contenus dans la formulation en une quantité allant jusqu'à 20 % en poids, de préférence jusqu'à 10 % en poids, encore plus préférentiellement jusqu'à 5 % en poids et le plus préférentiellement jusqu'à 2 % en poids.

7. Formulation pharmaceutique selon l'une quelconque des revendications 1 à 6, présentée sous forme de gélules, de capsules gastro-résistantes, de capsules gastro-résistantes, de capsules gelatinées molles, de comprimés ou de comprimés enrobés.

8. Procédé de préparation de la formulation pharmaceutique selon l'une quelconque des revendications 1 à 7, comprenant les étapes suivantes :
a) dissolution d'au moins un cannabinoïde choisi parmi le cannabidiol, le cannabigérol, le cannabinol, le D9-THC et le D8-THC, éventuellement avec des principes actifs lipophiles ou des substances actives auxiliaires, dans du monooléate de sorbitan polyoxyéthyléné ;
b) application du mélange obtenu à l'étape a) sur le sorbant poreux particulaire ;
c) homogénéisation du produit de l'étape b) ;
d) mélange éventuel du produit de l'étape c) avec des principes actifs hydrophiles ou des substances actives auxiliaires.

9. Formulation pharmaceutique selon l'une quelconque des revendications 1 à 7, pour utilisation comme médicament.

10. Formulation pharmaceutique selon l'une quelconque des revendications 1 à 7, pour utilisation pour la prévention et le traitement des troubles anxieux et des troubles du sommeil, pour le traitement de la schizophrénie, des troubles de stress post-traumatique, de la maladie de Parkinson, de la maladie d'Alzheimer, de la paraphrénie, du glaucome, pour la suppression de l'inflammation, pour la suppression des douleurs aiguës/chroniques/neuropathiques, pour le traitement des maladies auto-immunes, du diabète, de l'épilepsie, des inflammations intestinales idiopathiques, y compris la maladie de Crohn, pour le traitement des maladies cardiovasculaires, y compris le traitement de l'hypertension artérielle, pour le traitement des troubles et maladies gastro-intestinaux, pour le traitement de l'inflammation de la prostate et des affections après ablation de la prostate, comme traitement antitumoral adjuvant, pour la réduction du stress oxydatif, pour améliorer la qualité du sommeil, pour améliorer les effets cognitifs, et comme médicament antibactérien, antiviral et neuroprotecteur.
